(19) **Europäisches Patentamt / European Patent Office / Office européen des brevets**

(11) **EP 3 100 767 B1**

(12) **EUROPEAN PATENT SPECIFICATION**

(45) Date of publication and mention
of the grant of the patent:
**20.11.2019 Bulletin 2019/47**

(21) Application number: **14880395.0**

(22) Date of filing: **29.01.2014**

(51) Int Cl.:
*A61N 7/02* (2006.01)     *A61B 8/00* (2006.01)

(86) International application number:
**PCT/KR2014/000841**

(87) International publication number:
**WO 2015/115683 (06.08.2015 Gazette 2015/31)**

(54) **HIGH-INTENSITY FOCUSED ULTRASONIC WAVE TREATMENT DEVICE AND METHOD FOR CONTROLLING SAME**

HOCHINTENSIVE FOKUSSIERTE ULTRASCHALLWELLENBEHANDLUNGSVORRICHTUNG UND VERFAHREN ZUR STEUERUNG DAVON

DISPOSITIF DE TRAITEMENT PAR ONDES ULTRASONORES FOCALISÉES À HAUTE INTENSITÉ ET SON PROCÉDÉ DE COMMANDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.01.2014 KR 20140010666**

(43) Date of publication of application:
**07.12.2016 Bulletin 2016/49**

(73) Proprietor: **Alpinion Medical Systems Co., Ltd.**
**Seoul 152-848 (KR)**

(72) Inventors:
• **KIM, Dae-Seung**
**Seoul 157-882 (KR)**
• **KIM, Myung-Deok**
**Seoul 152-880 (KR)**
• **KANG, Kook-Jin**
**Yongin-si**
**Gyeonggi-do 448-536 (KR)**
• **SON, Keon-Ho**
**Seongnam-si**
**Gyeonggi-do 463-970 (KR)**

(74) Representative: **Brann AB**
**P.O. Box 3690**
**Drottninggatan 27**
**103 59 Stockholm (SE)**

(56) References cited:
EP-A1- 2 332 614     EP-A1- 2 481 447
WO-A1-2012/136786     KR-A- 20120 010 011
KR-A- 20120 091 737     KR-A- 20120 100 049
KR-A- 20130 055 972     KR-A- 20130 094 814
KR-A- 20130 106 361     US-A1- 2011 257 523

**Description**

[Technical Field]

The present invention relates to a high-intensity focused ultrasonic wave (HIFU) treatment device that is used for treatment using high temperature heat generated at a focus when high-intensity ultrasonic energy is concentrated on one region.

[Background Art]

High-Intensity Focused Ultrasonic Wave (HIFU) treatment is a medical technology that is provided to eliminate lesion tissues inside a body by burning the lesion tissues using high temperature heat of 65 to 100 degrees Celsius that is generated at a focus when high-intensity ultrasonic energy is concentrated on the one region. Specifically, when ultrasonic waves having an intensity one hundred thousand times stronger than that of ultrasonic waves used in diagnosis is concentrated on one region, heat is generated at a focus, and the heat is used to eliminate lesion tissues inside a body by burning them.

Since the heat is generated only at a focal point at which ultrasonic waves, which are harmless to the human body, are focused, lesions inside a human body are treated non-invasively. A HIFU treatment is available for pancreatic cancer, uterine fibroids, and liver cancer, and has been studied for prostate cancer, endometrial cancer, renal cancer, breast cancer, soft tissue tumors, and bone tumors.

A HIFU treatment device according to a conventional technology includes a HIFU transducer. The HIFU transducer is configured to focus high-intensity ultrasonic waves using a high-intensity ultrasonic wave emitting surface. The HIFU treatment device further includes an imaging transducer which obtains an image so that a diagnosis is made at a treatment region and a treatment process is monitored. The imaging transducer is provided as a one-dimensional array ultrasonic wave transducer having ultrasonic wave elements aligned on a single axis so that a two dimensional slice image is obtained.

In order to monitor a treatment process, an image plane of the imaging transducer needs to be aligned with a focal position of HIFUs steered by the HIFU transducer. To this end, an insertion hole is formed in the center of the high-intensity ultrasonic wave emitting surface in the HIFU transducer. The imaging transducer is operated to rotate with an ultrasonic wave transmission/reception portion thereof inserted into the insertion hole of the high-intensity ultrasonic wave emitting-surface. In a rotating process, the image plane of the imaging transducer is aligned with a focal position of HIFUs.

Meanwhile, in order to increase a lateral resolution of an image obtained by the imaging transducer such that image quality is improved, the ultrasonic wave transmission/reception portion of the imaging transducer is provided to have a large size. However, as the size of the ultrasonic wave transmission/reception portion of the imaging transducer is increased, the size of the insertion hole of the high-intensity ultrasonic wave emitting surface, into which the imaging transducer is inserted, is increased, and thus, the size of an ultrasonic wave emitting portion of the HIFU transducer is decreased. As a result, the HIFU transducer is provided with a lowered focal gain and a lowered resolution of HIFUs while having an increased side lobe, which causes the quality of an acoustic field of HIFUs to be degraded.

In order to remove the above-described constraints, when the size of the insertion hole of the high-intensity ultrasonic wave emitting surface is small and the size of the ultrasonic wave emitting portion of the HIFU transducer is large, since the HIFU transducer has an improved focal gain and an improved resolution of HIFUs while having a decreased side lobe, the quality of an acoustic field of HIFUs may be improved. However, as the size of the insertion hole of the high-intensity ultrasonic wave emitting surface is decreased, the size of the ultrasonic wave transmission/reception portion of the imaging transducer is decreased, and thus a lateral resolution of an image obtained by the imaging transducer is lowered and the image quality is degraded.

EP 2332614 discloses a device for treatment of an organ comprising at least one transducer for emission of HIFU waves, means for rotating the transducer and means for synchronizing the rotation of the transducer and an additional image transducer.

WO 2012/136786 discloses a probe comprising a therapy transducer and an imaging transducer wherein the imaging transducer has a rotation axis corresponding to the acoustic axis of the therapy transducer whereby the focal point of the therapy transducer is comprised in the imaging plane of the imaging transducer, the imaging transducer being fixed to the therapy transducer.

[Disclosure]

[Technical Problem]

The present invention is directed to providing a high-intensity focused ultrasonic wave (HIFU) treatment device capable of ensuring the quality of an image obtained by an imaging transducer while ensuring the quality of an acoustic field of HIFUs obtained by an HIFU transducer.

[Technical Solution]

One aspect of the present invention provides a high-intensity focused ultrasonic wave (HIFU) treatment device including a first HIFU transducer, a second HIFU transducer, an imaging transducer, a rotation driving part, and a controller. The first HIFU transducer emits HIFUs, and has an insertion hole formed at a center thereof with an insertion hole. The second HIFU transducer emits HIFUS and is inserted into the insertion hole of the first HIFU transducer and rotate therein. The imaging transducer is installed on the second HIFU transducer to rotate together with the second HIFU transducer and obtains an image. The rotation driving part rotates the second HIFU transducer. The controller controls the first and second HIFU transducers, the imaging transducer, and the rotation driving part.

Another aspect of the present invention provides a HIFU treatment device including a HIFU transducer, an imaging transducer, a rotation driving part, and a controller. The HIFU transducer emits HIFUs. The imaging transducer obtains an image and rotates with a portion thereof overlapping an ultrasonic wave emitting portion of the HIFU transducer at a center of the HIFU transducer. The rotation driving part rotates the imaging transducer. The controller controls the HIFU transducer, the imaging transducer, and the rotation driving part.

Another aspect of the present disclosure provides a method for controlling a HIFU treatment device. The method which is not part of the invention includes: aligning an image plane of an imaging transducer with a focal position of HIFUs by rotating a second HIFU transducer, on which the imaging transducer is installed and which is inserted into an insertion hole of a first HIFU transducer, relative to the first HIFU transducer; and focusing HIFUs on the focal position by driving the first and second HIFU transducers.

Another aspect of the present disclosure provides a method for controlling a HIFU treatment device. The method which is not part of the invention includes: aligning an image plane of an imaging transducer with a focal position of HIFUs by rotating an imaging transducer, which has a portion thereof overlapping an ultrasound emitting portion of a HIFU transducer at a center of the HIFU transducer, relative to the HIFU transducer; and focusing HIFUs on the focal position by driving the HIFU transducer.

[Advantageous Effects]

A high-intensity focused ultrasonic wave (HIFU) treatment device according to the present invention can improve the quality of an acoustic field of HIFUs by increasing an ultrasonic wave radiation area of the HIFUs. In addition, while sufficiently securing a radiation area of HIFUs, an ultrasonic wave transmission/reception portion have a large size so that image quality can be improved by increasing a lateral resolution of an image obtained by an imaging transducer.

[Description of Drawings]

FIG. 1 is a perspective view illustrating a high-intensity focused ultrasonic wave (HIFU) treatment device according to a first exemplary embodiment of the present invention.
FIGS. 2 and 3 are views for describing an example of operating the HIFU treatment device shown in FIG.1.
FIGS. 4 and 5 are views for describing an example of setting a focal position of HIFUs.
FIG. 6 is a perspective view illustrating a HIFU treatment device according to a second exemplary embodiment of the present invention.
FIG. 7 is an exploded perspective view of FIG. 6.
FIGS. 8 and 9 are views for describing an example of operating the HIFU treatment device shown in FIG.6.

[Modes of the Invention]

Hereinafter, exemplary embodiments of the present invention will be described with reference to accompanying drawings in detail. In the following description, the same elements will be assigned the same reference numerals throughout the drawings, and a detailed description of repeated descriptions and known functions and configurations incorporated herein will be omitted when they are deemed to obscure the subject matter of the present invention. The embodiments to be described below are provided so that the present invention is fully and completely described to those skilled in the art. Thus, the shapes and sizes of elements shown in the drawings may be exaggerated for the purpose of clear explanation.

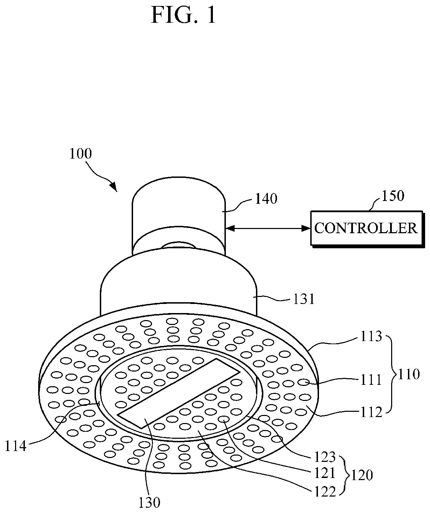

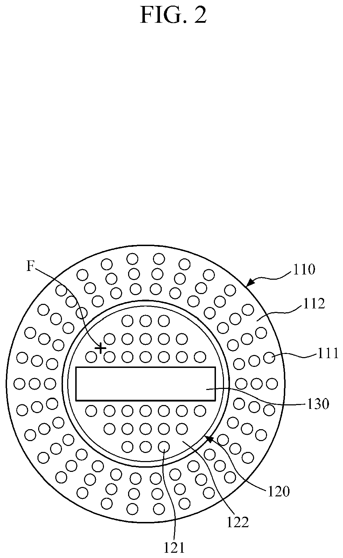

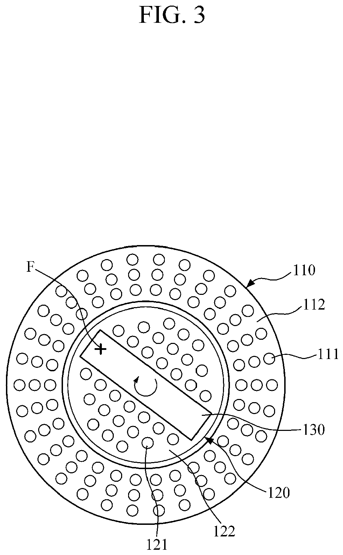

FIG. 1 is a perspective view illustrating a high-intensity focused ultrasonic wave (HIFU) treatment device according to a first exemplary embodiment of the present invention. FIGS. 2 and 3 are views for describing an example of operating the HIFU treatment device shown in FIG.1.

Referring to FIGS. 1 to 3, a HIFU treatment device 100 includes a first HIFU transducer 110, a second HIFU transducer 120, an imaging transducer 130, a rotation driving part 140, and a controller 150.

The first HIFU transducer 110 is configured to emit HIFUs for treating a patient. An insertion hole 114 is formed in the center of the first HIFU transducer 110.

For example, the first HIFU transducer 110 may include a plurality of first high-intensity ultrasonic wave vibrators 111 and a first high-intensity ultrasonic wave emitting surface 112. The first high-intensity ultrasonic wave vibrators 111 generate high-intensity ultrasonic waves. The first high-intensity ultrasonic wave vibrators 111 may be installed on the first high-intensity ultrasonic wave emitting surface 112 by being arranged thereon. The first high-intensity ultrasonic wave vibrators 111 may each include piezoelectric elements. The piezoelectric elements may resonate to generate ultrasonic waves when receiving a voltage from the controller 150. The first HIFU transducer 110 is implemented as a phased array transducer having a number of channels corresponding to the number of the first high-intensity ultrasonic wave vibrators 111.

The first high-intensity ultrasonic wave emitting surface 112 focuses high-intensity ultrasonic waves generated by the first high-intensity ultrasonic wave vibrators 111. The first high-intensity ultrasonic wave emitting surface 112 may be formed at a lower portion of a first support frame 113. The first support frame 113 may take the form of a circular plate. The insertion hole 114 may be provided at a center of the first support frame 113 in a circular shape. The first support frame 113 may be accommodated and fixed in a housing (not shown) having an open bottom side. Alternatively, the first support frame 113 may take the form of a hemisphere that is upwardly concave.

The second HIFU transducer 120 is configured to emit HIFUs. The second HIFU transducer 120 is inserted into the insertion hole 114 of the first HIFU transducer 110 and rotates. Accordingly, the second HIFU transducer 120 rotates inside of the first HIFU transducer 110. The imaging transducer 130 is installed on the second HIFU transducer 120.

For example, the second HIFU transducer 120 may include a plurality of second high-intensity ultrasonic wave vibrators 121 and a second high-intensity ultrasonic wave emitting surface 122. The second high-intensity ultrasonic wave vibrators 121 generate high-intensity ultrasonic waves. The second high-intensity ultrasonic wave vibrators 121 may be implemented in the same manner as the first high-intensity ultrasonic wave vibrators 111. The second high-intensity ultrasonic wave vibrators 121 may be installed on the second high-intensity ultrasonic wave emitting surface 122 by being arranged around the imaging transducer 130.

When the first HIFU transducer 110 is implemented as a phased array transducer, the second HIFU transducer 120 is also implemented as a phased array transducer having a number of channels corresponding to the number of the second high-intensity ultrasonic wave vibrators 121. The controller 150 controls phases of ultrasonic waves generated by the first and second high-intensity ultrasonic wave vibrators 111 and 121 to freely adjust a propagation direction and a focal distance of the ultrasonic waves. Accordingly, a focal position of HIFUs of the first and second HIFU transducers 110 and 120 may be adjusted.

The second high-intensity ultrasonic wave emitting surface 122 focuses high-intensity ultrasonic waves generated by the second high-intensity ultrasonic wave vibrators 121. The second high-intensity ultrasonic wave emitting surface 122 may be formed at a lower portion of a second support frame 123. When the first support frame 113 takes the form of a circular plate, the second support frame 123 may take the form of a circular plate. The second support frame 123 may have a size in which the second support frame 123 can be inserted into the insertion hole 114 while being spaced apart from the insertion hole 114 by a predetermined distance. The second support frame 123 may be fixed at a lower end of a casing 131 which accommodates the imaging transducer 130. The casing 131 may be rotated by the rotation driving part 140. Alternatively, when the first support frame 113 takes the form of a hemisphere, the second support frame 123 may also take the form of a hemisphere.

Although not shown, the first and second high-intensity ultrasonic wave emitting surfaces 112 and 122 may be covered by a membrane. A gap between the membrane and the first and second high-intensity ultrasonic wave emitting surfaces 112 and 122 may be filled with an ultrasonic wave transfer medium. The ultrasonic wave transfer medium may include deaerated water. HIFUs emitted by the first and second HIFU transducers 110 and 120 are transmitted to a lesion area of a patient via the ultrasonic wave transfer medium.

The imaging transducer 130 is provided to obtain an image. The imaging transducer 130 rotates together with the second HIFU transducer 120 by being installed on the second HIFU transducer 120. The imaging transducer 130 may obtain an image at each rotation position. The imaging transducer 130 may be controlled by the controller 150. The controller 150 may display the image obtained by the imaging transducer 130 through a display part of the HIFU treatment device 100.

For example, the imaging transducer 130 may be provided as a one-dimensional array ultrasonic wave transducer having ultrasonic wave elements arranged in a single axis so that a two-dimensional slice image is obtained. The ultrasonic wave elements may be implemented as piezoelectric elements. The piezoelectric elements resonate to generate an ultrasonic wave signal when receiving an electric signal, and vibrate to generate an electric signal when receiving an ultrasonic wave signal. The imaging transducer 130 may be protected by being accommodated in the casing 131.

The rotation driving part 140 rotates the second HIFU transducer 120. For example, the rotation driving part 140 may include a rotation motor. The rotation motor may be provided with a body fixed to the housing. The rotation motor may be fixed to the casing 131 with a driving shaft thereof positioned at a center of rotation of the second HIFU transducer 120. Accordingly, a rotation of the driving shaft of the rotation motor may rotate the second HIFU transducer 120.

The controller 150 may control the first and second HIFU transducers 110 and 120, the imaging transducer 130, and the rotation driving part 140. For example, referring to FIGS. 2 and 3, the controller 150 may control the rotation driving part 140 to rotate the second HIFU transducer 120 relative to the first HIFU transducer 110 so that an image plane of the imaging transducer 130 is aligned with a focal position F of HIFUs.

In this state, the controller 150 may update a position of each channel of the second HIFU transducer 120. The position of each channel of the second HIFU transducer 120 may represent a position of each of the second high-intensity ultrasonic wave vibrators 121. In this case, the controller 150 may calculate and update position coordinates of the respective second high-intensity ultrasonic wave vibrators 121 that are changed according to a rotation angle of the second HIFU transducer 120. Then, the controller 150 may control the first and second HIFU transducers 110 and 120 to focus high-intensity ultrasonic waves on the focal position F.

For example, coordinates of a center of each channel of the second HIFU transducer 120 which is changed according to a rotation angle of the second HIFU transducer 120 may be calculated and updated according to Equation 1 and Equation 2.

【Equation 1】

$$\theta = \operatorname{atan2}(y, x) = \begin{cases} \arctan\left(\dfrac{y}{x}\right) & x > 0 \\ \arctan\left(\dfrac{y}{x}\right) + \pi & y \geq 0, x < 0 \\ \arctan\left(\dfrac{y}{x}\right) - \pi & y < 0, x < 0 \\ +\dfrac{\pi}{2} & y > 0, x = 0 \\ -\dfrac{\pi}{2} & y < 0, x = 0 \\ \text{undefined} & y = 0, x = 0 \end{cases}$$

Herein, when coordinates of a focus are expressed based on an X-axis (a lateral axis), a Y-axis (an elevation axis), and a Z-axis (a depth axis), x and y represent a focal position in an X-axis direction and a focal position in a Y-axis direction, respectively, with respect to an origin positioned at the center of the second HIFU transducer. θ represents a rotation angle of the second HIFU transducer.

【Equation 2】

$$\begin{vmatrix} C X \\ C Y \end{vmatrix} = \begin{vmatrix} \cos\theta & -\sin\theta \\ \sin\theta & \cos\theta \end{vmatrix} \begin{vmatrix} C x \\ C y \end{vmatrix}$$

Herein, $Cx$ and $Cy$ represent coordinates of the center of each channel of the second HIFU transducer before the second HIFU transducer rotates. $CX$ and $CY$ represent coordinates of the center of each channel of the second HIFU transducer after the second HIFU transducer rotates by an angle of $\theta$.

The HIFU treatment device 100 includes not only a radiation area of HIFUs of the first HIFU transducer 110, but also a radiation area of HIFUs of the second HIFU transducer 120 around the imaging transducer 130. Accordingly, the HIFU treatment device 100 has a larger radiation area of HIFUs when compared to the conventional technology. In this manner, since the HIFU treatment device 100 has an improved focal gain of the first and second HIFU transducers 110 and 120, an improved resolution of HIFUs, and a reduced side lobe, the quality of an acoustic field of HIFUs may be improved.

In addition, even though an ultrasonic wave transmission/reception portion of the imaging transducer 130 is made to be large, the radiation area of HIFUs is sufficiently secured. Accordingly, since an increased number of ultrasonic wave elements of the imaging transducer 130 may be provided, the image quality is improved by increasing a lateral resolution of an image obtained by the imaging transducer 130.

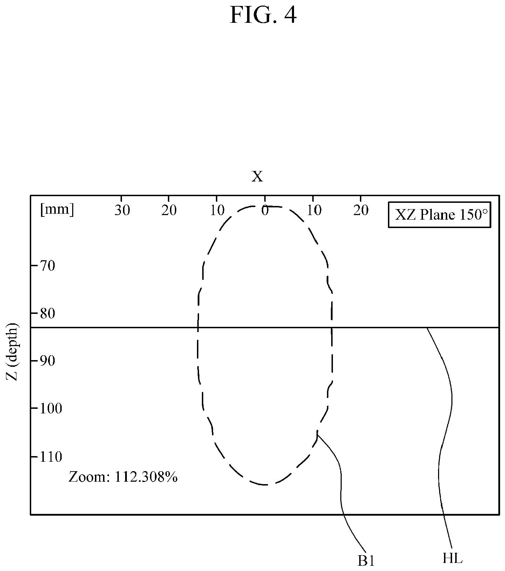

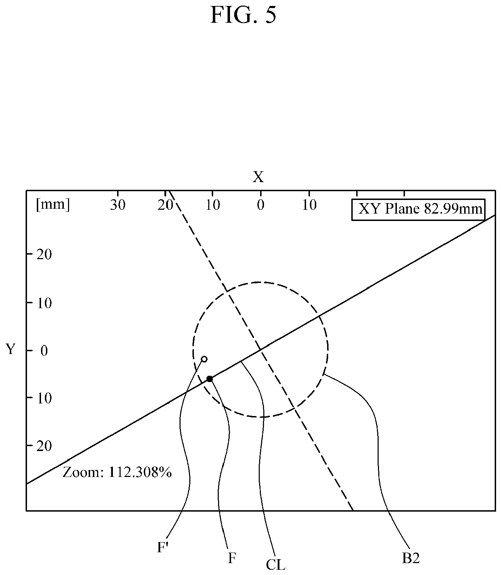

Hereinafter, a method for controlling the HIFU treatment device 100 will be described with reference to FIGS. 4 and 5 in conjunction with FIGS. 1 to 3. FIG. 4 is an XZ image plane obtained by the imaging transducer and displayed on the display part, and FIG. 5 is an XY image plane obtained by the imaging transducer and displayed on the display part.

First, an operator may position the HIFU treatment device 100 at a lesion area of a patient. In this case, the operator may preform a pre-scan on the lesion area of the patient while checking an image obtained by the imaging transducer 130 through the display part. Then, the operator may arrange the HIFU treatment device 100 such that the lesion area of the patient is positioned within a high-intensity ultrasonic wave steering range of the first and second HIFU transducers 110 and 120.

Then, the operator may set the focal position F of HIFUs to suit the lesion area of the patient while checking the image through the display part. Specifically, referring to FIG. 4, the operator manipulates an operating part of the HIFU treatment device 100 to input a horizontal line HL to correspond to a lesion area within a high-intensity ultrasonic wave steering range B1 displayed on the XZ image plane so that a focal depth in a Z-axis direction is set. Alternatively, the focal depth in the Z-axis direction may be set by inputting the horizontal line HL on a YZ image plane.

Then, as shown in FIG. 5, a high-intensity ultrasonic wave steering range B2 at the set focal depth is displayed on the XY image plane. The operator manipulates the operating part of the HIFU treatment device 100 to set the focal position F in the X-axis direction and the Y-axis direction within the high-intensity ultrasonic wave steering range B2 of the XY image plane. At this time, a plurality of focal positions F and F' may be set. In this case, the controller 150 may automatically make a schedule for sequentially rotating the second HIFU transducer 120 to the focal positions F and F'.

When the setting of the focal positions F and F' is completed, the operator manipulates the operating part so that the second HIFU transducer 120 is rotated relative to the first HIFU transducer 110. In this case, the second HIFU transducer 120 is controlled by the controller 150 to be rotated until an image plane of the imaging transducer 130 is aligned with one the focal position F of the focal positions of HIFUs. Then, the controller 150 may update the position of each channel of the second HIFU transducer 120 according to a rotation angle of the second HIFU transducer 120. Meanwhile, a check line CL that moves according to a rotation of the imaging transducer 130 is displayed on the XY image plane shown in FIG. 5. When the check line CL is positioned to pass through the focal position F, the operator confirms that the image plane of the imaging transducer 130 has been aligned with the focal position F of HIFUs.

In this state, the operator manipulates the operating part to drive the first and second HIFU transducers 110 and 120 while checking the image generated by the imaging transducer 130 through the display part. As the first and second HIFU transducers 110 and 120 are driven, high-intensity ultrasonic waves are focused on the focal position F.

Then, when the driving of the first and second HIFU transducers 110 and 120 stops, the second HIFU transducer 120 is rotated by the controller 150 so that the image plane of the imaging transducer 130 is aligned with the other focal position F'. Then, the operator manipulates the operating part to drive the first and second HIFU transducers 110 and 120 so that high-intensity ultrasonic waves are focused at the other focal position F' while checking the image generated by the imaging transducer 130 through the display part. In the process, the lesion area of the patient is treated by the high-intensity ultrasonic waves.

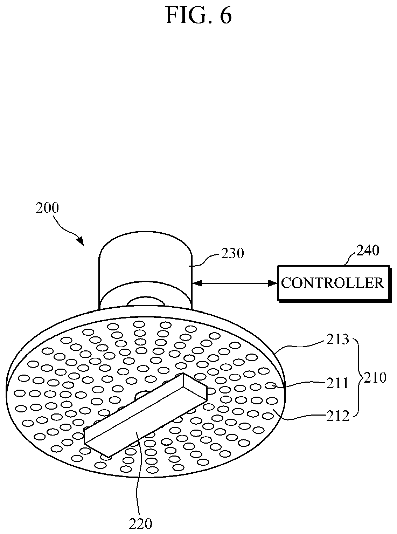

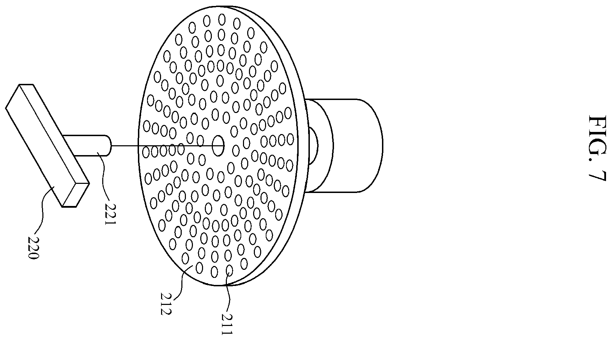

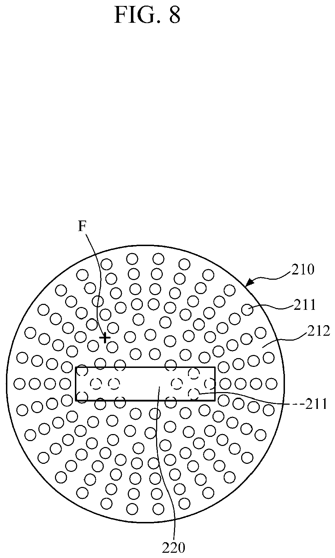

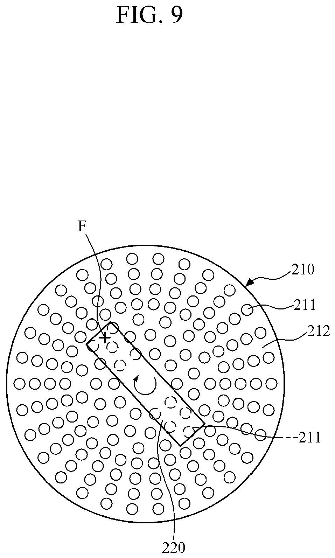

FIG. 6 is a perspective view illustrating a HIFU treatment device according to a second exemplary embodiment of the present invention. FIG. 7 is an exploded perspective view of FIG. 6. FIGS. 8 and 9 are views for describing an example of operating the HIFU treatment device shown in FIG.6.

Referring to FIGS. 6 to 9, a HIFU treatment device 200 includes a HIFU transducer 210, an imaging transducer 220, a rotation driving part 230, and a controller 240.

The HIFU transducer 210 is configured to emit HIFUs. The HIFU transducer 210 may include a plurality of high-intensity ultrasonic wave vibrators 211 and a high-intensity ultrasonic wave emitting surface 212. The high-intensity ultrasonic wave vibrators 211 generate high-intensity ultrasonic waves. The high-intensity ultrasonic wave vibrators 211 are installed on the high-intensity ultrasonic wave emitting surface 212 by being arranged thereon. Some of the high-intensity ultrasonic wave vibrators 211 are disposed to overlap the imaging transducer 220. The HIFU transducer 210 is implemented as a phased array transducer having a number of channels corresponding to the number of the high-intensity ultrasonic wave vibrators 211.

The high-intensity ultrasonic wave emitting surface 212 focuses high-intensity ultrasonic waves generated by the high-intensity ultrasonic wave vibrators 211. The high-intensity ultrasonic wave emitting surface 212 is formed at a lower portion of a support frame 213. The support frame 213 takes the form of a circular plate. A shaft hole through which a rotary shaft 221 of the imaging transducer 220 passes is formed at a center of the support frame 213. Alternatively, the support frame 213 may take the form of a hemisphere that is upwardly concave.

The imaging transducer 220 is provided to obtain an image. The imaging transducer 220 may be provided as a one-dimensional array ultrasonic wave transducer to obtain a two-dimensional slice image. The imaging transducer 220 rotates with one portion thereof overlapping an ultrasonic wave emitting portion of the HIFU transducer 210 at the center of the HIFU transducer 210. The imaging transducer 220 may rotate while facing the high-intensity ultrasonic wave emitting surface 212 of the HIFU transducer 210.

The rotation driving part 230 rotates the imaging transducer 220. The rotation driving part 230 may include a rotation motor. The rotation motor may have a driving shaft thereof fixed to the rotary shaft 221 of the imaging transducer 220 so that a rotation of the rotation motor may rotate the imaging transducer 220.

The controller 240 may control the HIFU transducer 210, the imaging transducer 220, and the rotation driving part 230. For example, referring to FIGS. 8 and 9, the controller 240 may control the rotation driving part 230 to rotate the imaging transducer 220 relative to the HIFU transducer 210 so that an image plane of the imaging transducer 220 is aligned with a focal position of HIFUs. In this state, the controller 240 turns off some channels overlapping the imaging transducer 220 among the channels of the HIFU transducer 210 and turns on the remaining channels to focus high-intensity ultrasonic waves on the focal position.

The position of each channel of the HIFU transducer 210 may represent a position of each of the high-intensity ultrasonic wave vibrators 211. In this case, the controller 240 may identify positions of some of the high-intensity ultrasonic wave vibrators 211 which overlap the imaging transducer 220 according to a rotation angle of the imaging transducer 220 and turn off the high-intensity ultrasonic wave vibrators 211 having the identified positions. Accordingly, not only the imaging transducer 220 but also the high-intensity ultrasonic wave vibrators 211 overlapping the imaging transducer 220 are prevented from being damaged during the driving of the HIFU transducer 210.

Similar to the HIFU treatment device 100 according to the above-described embodiment, the HIFU treatment device 200 according to the second exemplary embodiment of the present invention may improve the quality of an acoustic field of HIFUs by having a larger radiation area of HIFUs. In addition, an increased number of ultrasonic wave elements of the imaging transducer 220 may be provided so that image quality is improved by increasing a lateral resolution of an image obtained by the imaging transducer 220.

A method for controlling the HIFU treatment device 200 will be described with reference to FIGS. 6 to 9.

First, an operator may position the HIFU treatment device 200 at a lesion area of a patient. In this case, the operator may perform a pre-scan on the lesion area of the patient while checking an image obtained by the imaging transducer 220 through a display part. Then, the operator may arrange the HIFU treatment device 200 so that the lesion area of the patient is positioned within a high-intensity ultrasonic wave steering range of the HIFU transducer 210.

Then, the operator may manipulate an operating part of the HIFU treatment device 200 to set the focal position F of HIFUs to suit the lesion area of the patent while checking the image through the display part. Then, the operator manipulates the operating part so that the imaging transducer 220 is rotated relative to the HIFU transducer 210. In this case, the imaging transducer 220 is controlled to be rotated until an image plane of the imaging transducer 220 is aligned with the focal position F of HIFUs. In the process, the controller 240 may identify positions of channels of the HIFU transducer 210 which overlap the imaging transducer 220 according to a rotation angle of the imaging transducer 220.

When the image plane of the imaging transducer 220 is aligned with the focal position F of HIFUs, the operator drives the HIFU transducer 210 by manipulating the operating part while checking the image generated by the imaging transducer 220 through the display part. At this time, the controller 240 turns off some channels overlapping the imaging transducer 220 among the channels of the HIFU transducer 210 and turns on the remaining channels. According to the driving of the HIFU transducer 210, high-intensity ultrasonic waves are focused on the focal position F so that the lesion area of the patient is treated.

While the invention has been described with reference to exemplary embodiments illustrated in accompanying drawings, these should be considered in a descriptive sense only, and it will be understood by those skilled in the art that various alterations and equivalent other embodiment may be made. Therefore, the scope of the invention is defined by the appended claims.

**Claims**

1. A high-intensity focused ultrasonic wave (HIFU) treatment device (100) comprising:

   a first HIFU transducer (110) configured to emit HIFUs, and having an insertion hole (114) formed at a center thereof;
   a second HIFU transducer (120) configured to emit HIFUs and to be inserted into the insertion hole of the first HIFU transducer and rotate therein;
   an imaging transducer (130) installed on the second HIFU transducer to rotate together with the second HIFU transducer and configured to obtain an image;
   a rotation driving part (140) configured to rotate the second HIFU transducer; and
   a controller (150) configured to control the first and second HIFU transducers (110, 120), the imaging transducer (130), and the rotation driving part (140).

2. The HIFU treatment device of claim 1, wherein the controller (150) controls the rotation driving part (140) to rotate the second HIFU transducer (120) relative to the first HIFU transducer (110) so that an image plane of the imaging transducer (130) is aligned with a focal position (F) of the HIFUs.

3. The HIFU treatment device of claim 2, wherein the controller (110), updates a position of each channel of the second HIFU transducer (120) when the image plane of the imaging transducer (130) is aligned with the focal position of HIFUs, and subsequently controls the first and second HIFU transducers (110, 120) so that high-intensity ultrasonic waves are focused on the focal position (F),
   wherein the each channel of the second HIFU transducer (120) corresponds to respective second high-intensity ultrasonic wave vibrator (121).

4. The HIFU treatment device of claim 1, wherein the first and second HIFU transducers (110, 120) are phase array transducers.

5. The HIFU treatment device of claim 1, wherein the imaging transducer (130) is provided as a one-dimensional array ultrasonic wave transducer having ultrasonic wave elements arranged in a single axis.

6. A high-intensity focused ultrasonic wave (HIFU) treatment device (200) comprising:

   a HIFU transducer (210) configured to emit HIFUs;
   an imaging transducer (220) configured to obtain an image and to rotate with a portion thereof overlapping an ultrasonic wave emitting portion of the HIFU transducer (210) at a center of the HIFU transducer;
   a rotation driving part (230) configured to rotate the imaging transducer (220); and
   a controller (240) configured to control the HIFU transducer (210), the imaging transducer (220), and the rotation driving part (230), **characterized in that:**

   the controller (240) controls the rotation driving part (230) to rotate the imaging transducer (220) relative to the HIFU transducer (210) so that an image plane of the imaging transducer (220) is aligned with a focal position (F) of HIFUs obtained by the HIFU transducer (210), and
   the controller (240) turns off channels of the HIFU transducer (210) overlapping the imaging transducer (220) and turns on remaining channels of the HIFU transducer (210) to focus high-intensity ultrasonic waves on the focal position (F) when the image plane of the imaging transducer (220) is aligned with the focal position of HIFUs,
   wherein the channels of the HIFU transducer (210) correspond to high-intensity ultrasonic wave vibrators (211).

7. The HIFU treatment device of claim 6, wherein the HIFU transducer (210) is a phase array transducer.

8. The HIFU treatment device of claim 6, wherein the imaging transducer (210) is provided as a one-dimensional array ultrasonic wave transducer having ultrasonic wave elements arranged in a single axis.

**Patentansprüche**

1. Behandlungsvorrichtung mit hochintensiven fokussierten Ultraschallwellen (HIFU) (100), umfassend:

   einen ersten HIFU-Wandler (110), der konfiguriert ist, um HIFUs zu emittieren, und ein in einer Mitte davon gebildetes Einführloch (114) aufweist;
   einen zweiten HIFU-Wandler (120), der konfiguriert ist, um HIFUs zu emittieren und in das Einführloch des ersten HIFU-Wandlers eingeführt zu werden und sich darin zu drehen;
   einen Bildgebungswandler (130), der an dem zweiten HIFU-Wandler angebracht ist, um sich gemeinsam mit dem zweiten HIFU-Wandler zu drehen, und konfiguriert ist, um ein Bild zu erhalten;
   ein Drehantriebsteil (140), das konfiguriert ist, um den zweiten HIFU-Wandler zu drehen; und
   eine Steuerung (150), die konfiguriert ist, um den ersten und zweiten HIFU-Wandler (110, 120), den Bildgebungswandler (130) und das Drehantriebsteil (140) zu steuern.

2. HIFU-Behandlungsvorrichtung nach Anspruch 1, wobei die Steuerung (150) das Drehantriebsteil (140) steuert, um den zweiten HIFU-Wandler (120) relativ zu dem ersten HIFU-Wandler (110) so zu drehen, dass eine Bildebene des Bildgebungswandlers (130) an einer Fokusposition (F) der HIFUs ausgerichtet wird.

3. HIFU-Behandlungsvorrichtung nach Anspruch 2, wobei die Steuerung (110) eine Position jedes Kanals des zweiten HIFU-Wandlers (120) aktualisiert, wenn die Bildebene des Bildgebungswandlers (130) an der Fokusposition der HIFUs ausgerichtet wird, und nachfolgend den ersten und zweiten HIFU-Wandler (110, 120) so steuert, dass hochintensive fokussierte Ultraschallwellen auf die Fokusposition (F) fokussiert werden,
   wobei jeder Kanal des zweiten HIFU-Wandlers (120) einem jeweiligen zweiten hochintensiven Ultraschallwellenvibrator (121) entspricht.

4. HIFU-Behandlungsvorrichtung nach Anspruch 1, wobei der erste und zweite HIFU-Wandler (110, 120) Phasenarray-Wandler sind.

5. HIFU-Behandlungsvorrichtung nach Anspruch 1, wobei der Bildgebungswandler (130) als eindimensionaler Array-Ultraschallwellenwandler, der in einer einzigen Achse angeordnete Ultraschallwellenelemente aufweist, bereitgestellt wird.

6. Behandlungsvorrichtung mit hochintensiven fokussierten Ultraschallwellen (HIFU) (200), umfassend:

   einen HIFU-Wandler (210), der konfiguriert ist, um HIFUs zu emittieren;
   einen Bildgebungswandler (220), der konfiguriert ist, um ein Bild zu erhalten und sich gemeinsam mit einem Abschnitt davon, der sich mit einem Ultraschallwellen emittierenden Abschnitt des HIFU-Wandlers (210) in einer Mitte des HIFU-Wandlers überschneidet, zu drehen;
   ein Drehantriebsteil (230), das konfiguriert ist, um den Bildgebungswandler (220) zu drehen; und
   eine Steuerung (240), die konfiguriert ist, um den HIFU-Wandler (210), den Bildgebungswandler (220) und das Drehantriebsteil (230) zu steuern, **dadurch gekennzeichnet, dass:**

      die Steuerung (240) das Drehantriebsteil (230) steuert, um den Bildgebungswandler (220) relativ zu dem HIFU-Wandler (210) so zu drehen, dass eine Bildebene des Bildgebungswandlers (220) an einer Fokusposition (F) der mittels des HIFU-Wandlers (210) erhaltenen HIFUs ausgerichtet wird, und
      die Steuerung (240) Kanäle des HIFU-Wandlers (210), die sich mit dem Bildgebungswandler (220) überschneiden, abschaltet und verbleibende Kanäle des HIFU-Wandlers (210) anschaltet, um hochintensive Ultraschallwellen auf die Fokusposition (F) zu fokussieren, wenn die Bildebene des Bildgebungswandlers (220) an der Fokusposition der HIFUs ausgerichtet wird,
      wobei die Kanäle des HIFU-Wandlers (210) hochintensiven Ultraschallwellenvibratoren (211) entsprechen.

7. HIFU-Behandlungsvorrichtung nach Anspruch 6, wobei der HIFU-Wandler (210) ein Phasenarray-Wandler ist.

8. HIFU-Behandlungsvorrichtung nach Anspruch 6, wobei der Bildgebungswandler (210) als eindimensionaler Array-Ultraschallwellenwandler, der in einer einzigen Achse angeordnete Ultraschallwellenelemente aufweist, bereitgestellt wird.

**Revendications**

1. Dispositif de traitement (100) par ondes ultrasonores focalisées à haute intensité (HIFU) comprenant :

   un premier transducteur d'HIFU (110) configuré pour émettre des HIFU, et ayant un trou d'insertion (114) formé en son centre ;
   un second transducteur d'HIFU (120) configuré pour émettre des HIFU et pour être inséré dans le trou d'insertion du premier transducteur d'HIFU et tourner dans celui-ci ;
   un transducteur d'imagerie (130) installé sur le second transducteur d'HIFU pour tourner conjointement avec le second transducteur d'HIFU et configuré pour obtenir une image ;
   une partie d'entraînement en rotation (140) configurée pour faire tourner le second transducteur d'HIFU ; et
   un dispositif de commande (150) configuré pour commander les premier et second transducteurs d'HIFU (110, 120), le transducteur d'imagerie (130), et la partie d'entraînement en rotation (140).

2. Dispositif de traitement par HIFU selon la revendication 1, dans lequel le dispositif de commande (150) commande la partie d'entraînement en rotation (140) pour faire tourner le second transducteur d'HIFU (120) par rapport au premier transducteur d'HIFU (110) de sorte qu'un plan d'image du transducteur d'imagerie (130) soit aligné avec une position focale (F) des HIFU.

3. Dispositif de traitement par HIFU selon la revendication 2, dans lequel le dispositif de commande (110) actualise une position de chaque canal du second transducteur d'HIFU (120) lorsque le plan d'image du transducteur d'imagerie (130) est aligné avec la position focale des HIFU, et commande ensuite les premier et second transducteurs d'HIFU (110, 120) de sorte que des ondes ultrasonores de haute intensité soient focalisées sur la position focale (F), dans lequel chaque canal du second transducteur d'HIFU (120) correspond à un second vibrateur à ondes ultrasonores focalisées à haute intensité respectif (121).

4. Dispositif de traitement par HIFU selon la revendication 1, dans lequel les premier et second transducteurs d'HIFU (110, 120) sont des transducteurs de réseau en phase.

5. Dispositif de traitement par HIFU selon la revendication 1, dans lequel le transducteur d'imagerie (130) est fourni sous la forme d'un transducteur d'ondes ultrasonores d'un réseau monodimensionnel ayant des éléments d'ondes ultrasonores agencés dans un axe unique.

6. Dispositif de traitement (200) par ondes ultrasonores focalisées à haute intensité (HIFU) comprenant :

   un transducteur d'HIFU (210) configuré pour émettre des HIFU ;
   un transducteur d'imagerie (220) configuré pour obtenir une image et pour tourner avec une portion de celui-ci chevauchant une portion émettant des ondes ultrasonores du transducteur d'HIFU (210) à un centre du transducteur d'HIFU ;
   une partie d'entraînement en rotation (230) configurée pour faire tourner le transducteur d'imagerie (220) ; et
   un dispositif de commande (240) configuré pour commander le transducteur d'HIFU (210), le transducteur d'imagerie (220), et la partie d'entraînement en rotation (230), **caractérisé en ce que** :

      le dispositif de commande (240) commande la partie d'entraînement en rotation (230) pour faire tourner le transducteur d'imagerie (220) par rapport au transducteur d'HIFU (210) de sorte qu'un plan d'image du transducteur d'imagerie (220) soit aligné avec une position focale (F) des HIFU obtenue par le transducteur d'HIFU (210), et
      le dispositif de commande (240) désactive des canaux du transducteur d'HIFU (210) chevauchant le transducteur d'imagerie (220) et active des canaux restants du transducteur d'HIFU (210) pour focaliser des ondes ultrasonores à haute intensité sur la position focale (F) lorsque le plan d'image du transducteur d'imagerie (220) est aligné avec la position focale des HIFU, dans lequel les canaux du transducteur d'HIFU (210) correspondent à des vibrateurs d'ondes ultrasonores à haute intensité (211).

7. Dispositif de traitement par HIFU selon la revendication 6, dans lequel le transducteur d'HIFU (210) est un transducteur de réseau en phase.

8. Dispositif de traitement par HIFU selon la revendication 6, dans lequel le transducteur d'imagerie (210) est fourni sous la forme d'un transducteur d'ondes ultrasonores d'un réseau monodimensionnel ayant des éléments d'ondes ultrasonores agencés dans un axe unique.

# FIG. 1

# FIG. 2

FIG. 3

# FIG. 4

FIG. 5

FIG. 6

FIG. 7

211

212

221

220

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2332614 A

- WO 2012136786 A